(19) Europäisches Patentamt
European Patent Office
Office européen des brevets

(11) Publication number : **0 493 042 A2**

(12) **EUROPEAN PATENT APPLICATION**

(21) Application number : **91311904.6**

(51) Int. Cl.$^5$ : **G01N 33/553**

(22) Date of filing : **20.12.91**

(30) Priority : **20.12.90 GB 9027607**

(43) Date of publication of application : **01.07.92 Bulletin 92/27**

(84) Designated Contracting States : **AT BE CH DE DK ES FR GB GR IT LI LU NL SE**

(71) Applicant : **OXFORD POLYTECHNIC
Gipsy Lane
Headington, Oxford OX3 OBP (GB)**

(72) Inventor : **Hutchins, Michael G., c/o Oxford Polytechnic
School of Engineering, Gipsy Lane, Headington
Oxford OX3 0BP (GB)**
Inventor : **McMeeking, Graham, c/o Oxford Polytechnic
School of Engineering, Gipsy Lane, Headington
Oxford OX3 0BP (GB)**

(74) Representative : **Pennant, Pyers et al
Stevens, Hewlett & Perkins 1 Serjeants' Inn
Fleet Street
London EC4Y 1LL (GB)**

(54) **Biosensor.**

(57) A method of assaying an analyte in a fluid sample by the use of a substrate whose surface is coated with an electrochromic film, comprises contacting the sample with the coated surface under conditions to cause a reagent to be deposited on the surface in a manner related to the presence of the analyte in the sample, and observing optical properties of the electrochromic film as an indication of the analyte in the sample.

EP 0 493 042 A2

This invention concerns an assay method and device comprising a substrate having a surface coated with an electrochromic film. The invention is based on the discovery that the optical properties of the electrochromic film are substantially changed by the presence of a thin coating on the electrochromic film.

Electrochromism is defined as reversible optical switching of a material to a coloured, bleached or intermediate state as a result of dual ion and electron insertion/removal under a low applied voltage. As a result of this injection/ejection, colour centres are formed in the material, producing optical absorption principally in the visible wavelength region, although near infra-red absorption is possible. These changes in optical density, between the coloured and bleached states, represent both chemical and structural changes in the material.

The most widely studied electrochromic material is tungsten oxide, which exhibits a transparent to blue colouration. However, there are other materials based on transition metal oxides, such as hydrated nickel oxide, that demonstrate much promise.

The subject is covered in a book entitled "Large Area Chromogenics: Materials and Devices for Transmittance Control" by C. M Lampert and C. G. Granqvist, Optical Engineering Press, Bellingham, WA, 1989. The chapter by C. M. Lampert entitled "Chemical and Optical Studies of Electrochromic Hydrated Nickel Oxide Films and Devices" is of particular interest.

There is controversy about the colouration mechanism in hydrated nickel oxide films. This is due to the facts that different methods of fabrication ultimately produce different basic structures and film densities, and that the films can be crystalline, amorphous or mixed.

Most of the nickel oxide films that are deposited by physical vapour deposition have to be cycled in aqueous potassium hydroxide in order to observe electrochromism. This may lead to the nickel oxide being partly converted to nickel hydroxide. The overall reaction can be written:

$$\underset{\text{(bleached)}}{Ni(OH)_2 + OH^-} \rightleftharpoons \underset{\text{(coloured)}}{NiOOH + e^- + H_2O}$$

The precise chemical state of the film is not material to the present invention. For convenience and brevity, the term nickel hydroxide film is used herein.

Biosensors have been proposed based on various detection phenomena, including surface plasmon resonance and Brewster angle reflectance. But the sensitivities of these detection phenomena are scarcely sufficient for the small changes involved in biological assays.

In one aspect, this invention provides a method of assaying an analyte in a fluid sample by the use of a substrate whose surface is coated with an electrochromic film, which method comprises contacting the sample with the coated surface under conditions to cause a reagent to be deposited on the surface in a manner related to the presence of the analyte in the sample, and observing optical properties of the electrochromic film as an indication of the analyte in the sample.

The electrochromic film is preferably of an anodically coloured metal hydroxide, for example of manganese or cobalt or iridium or preferably nickel. The substrate is conveniently made transparent e.g. of glass, preferably coated with an electrically conducting indium tin oxide or fluorine-doped tin oxide layer.

The electrochromic film can be deposited by a convenient technique. Known techniques for depositing nickel oxide/hydroxide films include physical vapour deposition by electron beam evaporation or sputtering; electrochemical deposition by anodic or cathodic techniques; and chemical deposition in separate or combined solutions of nickel sulphate and alkali. The assay method of the invention involves contacting a fluid sample with the coated substrate under conditions to cause a preferably macromolecular reagent to be deposited on the coated surface in a manner related to the presence of the analyte in the sample. The reagent may be, but is not necessarily, the analyte. The assay may be qualitative, i.e. detecting the presence of the analyte, or quantitative, i.e. determining its concentration. The assay may be performed on an equilibrium (i.e. determining extent of deposition) or a kinetic (i.e. determining rate of deposition) basis. The nature of the assay system is not material to the invention. Many systems are well known in the assay field, for example:-

a) In the simplest case, a fluid sample contains a macromolecular analyte, which becomes deposited on the electrochromic film and alters its optical properties.

b) When the analyte is an antibody, the electrochromic film is first coated with a hapten or antigen capable of binding the antibody. When a sample containing the analyte is contacted with the surface, the analyte becomes immobilised on the surface. As an alternative, when the analyte is a macromolecular antigen, the reagent pre-deposited on the coated surface can be an associated antibody.

c) Competition Assay Format. An analogue of the analyte is pre-deposited on the coated surface. A specific binder for the analyte is provided in solution. When a sample containing the analyte is contacted with the coated surface, analyte and analyte analogue compete for binding to the specific binder. The extent of deposition of the specific binder on the coated surface is related to the concentration of the analyte in the sample. This assay format is convenient when the analyte is a small molecule.

d) Sandwich Assay Format. Two different antibodies to the analyte are provided, and one is pre-deposited on the coated surface. When a sample containing the analyte is contacted with the coated surface, the analyte becomes immobilised on it. Then the other antibody in solution is contacted with the coated surface, and becomes immobilised on it to an extent related to the concentration of the analyte in the sample. This format may be useful to increase assay sensitivity.

Techniques for pre-depositing reagents on solid surfaces, whether by physical or chemical deposition, are well known in the art and will not be described here. It is preferred that the electrochromic film on the substrate be bleached prior to application of a reagent.

As a result of the above assay steps, the coated substrate carries a reagent in a manner (to an extent) related to the presence (concentration) of the analyte in the sample. In order to observe the optical properties of the electrochromic film, the coated substrate may be transferred to another preferably alkaline fluid medium. The pH should be high enough to permit colouration (of the uncovered film) but not so high as to denature or decompose the assay reagents. In this state, the electrochromic film can be coloured by being made anodic, or bleached by being made cathodic, using for the purpose the electrically conducting doped tin oxide layer on the substrate. Colouration may typically be achieved by a voltage below +1 V, e.g. +0.3 to +0.6 V relative to a standard calomel electrode.

The invention is based on the discovery that electrochromic films which carry a macromolecular coating on their surface have a very much reduced ability to be coloured by this treatment. It is presently believed that the macromolecular layer is acting as a simple physical barrier and reducing the rate or extent of diffusion to and from the bulk solutions of ions particularly hydroxyl ions required for colouration. The thicker or more complete or less porous is the macromolecular layer, the less is the ability of the underlying electrochromic film to be coloured.

In another aspect the invention provides a biosensor, that is to say a substrate having a surface coated with an electrochromic film and comprising on the coated surface a reagent for binding with and immobilising an analyte present in a fluid sample.

In yet another aspect, the invention provides an assay kit for assaying an analyte, comprising the coated substrate as defined and any other liquid phase reagents required for the assay e.g. to be mixed with a fluid sample containing the analyte.

The following example illustrates the invention. Reference is made to the accompanying drawing, which is a graph of percent transmittance against wavelength for an electrochromic film at various stages in the assay of an analyte. Detection of change can be made using broad-band technique or at a single wavelength in either the visible or near infrared spectral ranges.

## EXAMPLE

The substrate material used for the biosensor device was fluorine doped tin oxide glass. This was cleaned to remove dust and grease by using a sonicator bath and an aqueous solution containing 10% by mass of Decon 90 detergent. The substrate was then thoroughly washed with distilled water to remove detergent from the substrate. The substrate was then thoroughly dried using a nitrogen jet.

A 250 $cm^3$ aqueous solution containing 0.025 moles of $NiSO_4$ (aq) and 0.025 moles of $NH_4OH$ (aq) was prepared. The substrate was dipped into this solution and cycled between +1.5 V and -0.5 V at 20 mV/sec for 10 minutes. After deposition the film was washed in distilled water and dried using a nitrogen jet. The thickness of the film was evaluated by weight difference. This was determined to be 600 Å ± 100 Å.

The film was then bleached in a 1 molar solution of KOH (aq) at a potential of -0.5 V for 2 minutes. The film was removed from the KOH (aq) solution and washed using distilled water, followed by drying in a nitrogen jet. A measurement of the optical properties of the bleached film was made using a Beckman UV/visible/IR spectrophotometer.

A solution of HSA antigen of concentration 10g/litre was prepared. This was used to uniformly coat the whole of the bleached electrochromic film using a dropper pipette. The nickel hydroxide film + antigen was then left for 30 minutes to deposit a monolayer of antigen. After deposition the remaining liquid was removed using distilled water followed by drying in a nitrogen jet. The optical properties of the resulting double layer was measured.

Finally an aqueous solution of a HSA antibody was prepared at a concentration of 40 mg/litre. This was transferred to the previously prepared double layer such that it covered half of the area of the double layer. The film was left for 30 minutes. The remaining solution was washed off with distilled water and the 'triple' layer was dried using a nitrogen jet. The optical properties of the bleached nickel oxide, antigen and antibody layer

were measured.

Each time the device was electrochemically switched to the coloured state in a KOH bath by making the fluorine doped substrate positively charged at a voltage of +0.5 V for 2 minutes. The optical properties of the coloured nickel oxide were determined; with the results shown in Figure 1:-

Trace 1 is the nickel hydroxide film in the coloured state as initially prepared.

Trace 2 is the nickel hydroxide film coated with antigen in its coloured state.

Trace 3 is the nickel hydroxide film coated with antigen and then with antibody, again in its coloured state.

Trace 4 is the nickel hydroxide film, coated with antigen and then with antibody, in its bleached state.

Trace 5 is the nickel hydroxide film coated with antigen in its bleached state.

By comparing trace 2 with trace 1, it is clear that the antigen reduces the ability of the electrochromic film to be coloured.

By comparing trace 3 with traces 2 and 1, it is clear that the antibody further reduces the ability of the electrochromic film to be coloured.

By comparing traces 3, 4 and 5, it is apparent that the antibody has indeed completely destroyed the ability of the electrochromic film to be coloured.

Physical transmittance figures (obtained using a colouration voltage of +0.75 V instead of +0.5 V were as follows:-

1 - 25%
2 - 40%
3 - 74%
4 - 78%
5 - 79%

The large differences between these figures 1, 2 and 3 is an indication of the great sensitivity of the device.

## Claims

1. A method of assaying an analyte in a fluid sample by the use of a substrate whose surface is coated with an electrochromic film, which method comprises contacting the sample with the coated surface under conditions to cause a reagent to be deposited on the surface in a manner related to the presence of the analyte in the sample, and observing optical properties of the electrochromic film as an indication of the analyte in the sample.

2. A method as claimed in Claim 1, wherein the electrochromic film is of an anodically coloured metal hydroxide.

3. A method as claimed in Claim 2, wherein the anodically coloured metal hydroxide is nickel hydroxide.

4. A method as claimed in any one of Claims 1 to 3, wherein the electrochromic film is contacted with an aqueous alkaline solution in order to observe the optical properties.

5. A method as claimed in any one of Claims 1 to 4, wherein the substrate is transparent.

6. A method as claimed in any one of Claims 1 to 5, wherein the reagent is macromolecular.

7. A substrate having a surface coated with an electrochromic film and comprising on the coated surface a reagent for binding with and immobilising an analyte present in a fluid sample.

8. An assay kit for assaying an analyte, comprising the coated substrate of Claim 7 and any other liquid phase reagent required to be mixed with a fluid sample containing the analyte.

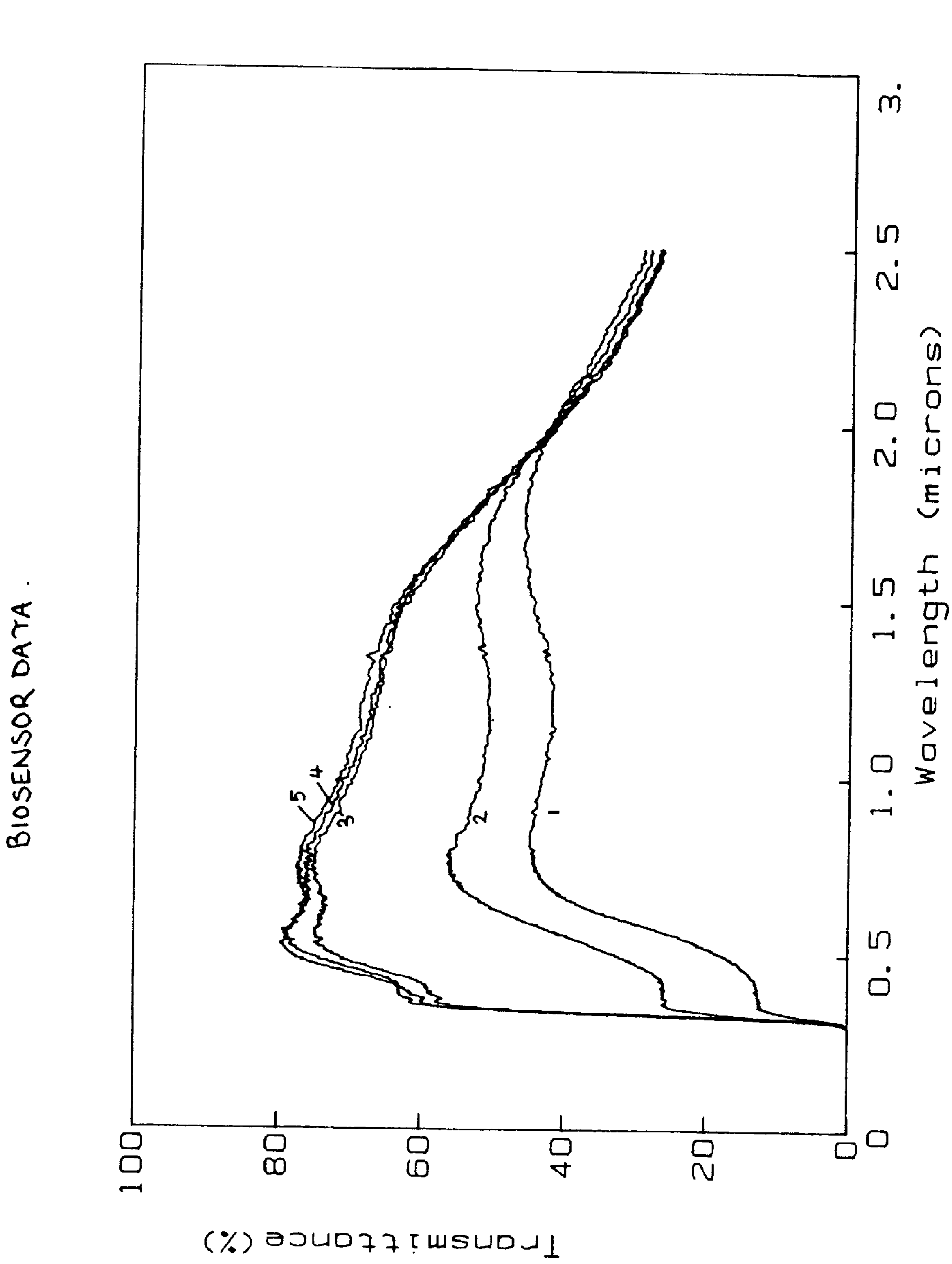
BIOSENSOR DATA.
Transmittance (%)
Wavelength (microns)
0
20
40
60
80
100
0
0.5
1.0
1.5
2.0
2.5
3.
1
2
3
4
5